# EUROPEAN PATENT APPLICATION

(11) **EP 4 484 401 A1**
(43) Date of publication of application: **01.01.2025**
(21) Application number: 23759974.1
(22) Date of filing: 21.02.2023
(51) Int. Cl.: C07C 29/46, C07C 33/02

(54) **PRODUCTION METHOD OF 2,7-OCTADIEN-1-OL**

(30) Priority: 25.02.2022 JP 2022028310
(71) Applicant: Kuraray Co., Ltd., Kurashiki-shi, Okayama 710-0801 (JP)
(72) Inventor: HOSAKA, Yusaku, Kamisu-shi, Ibaraki 314-0197 (JP); KOBAYASHI, Shohei, Kamisu-shi, Ibaraki 314-0197 (JP)
(74) Representative: D Young & Co LLP
(86) International application number: PCT/JP2023/006231
(87) International publication number: WO 2023/162973

(57) **Abstract**

Provided is a method for producing 2,7-octadien-1-ol, including a step (1) of feeding a raw material mixture liquid containing butadiene, a Group 6 to 11 transition metal catalyst containing a Group 6 to 11 transition metal, a tertiary phosphorus compound, an amine compound, and water to a telomerization reactor, and a step (2) of reacting butadiene and water in a carbon dioxide atmosphere in the telomerization reactor to obtain a reaction mixture liquid containing 2,7-octadien-1-ol, in which a residence time of the raw material mixture liquid in the telomerization reactor is 1.3 to 3.0 hours.

## Description

### Technical Field

The present invention relates to a method for producing 2,7-octadien-1-ol, and particularly to a method for producing 2,7-octadien-1-ol by reacting butadiene with water in the presence of a Group 6 to 11 transition metal catalyst containing a Group 6 to 11 transition metal.

### Background Art

2,7-Octadien-1-ol can be derived to 7-octenal by isomerization reaction, and 7-octenal can be derived to 1,9-nonanedial by hydroformylation reaction. Since the 1,9-nonanedial can be derived to 1,9-nonanediamine useful as a raw material for a polymer monomer by a reductive amination reaction, 2,7-octadien-1-ol has high industrial value, and development of a method for producing the same is also important.

As a method for producing 2,7-octadien-1-ol by a telomerization reaction of butadiene and water in the presence of a palladium catalyst composed of a palladium compound and an organic phosphorus compound, carbon dioxide and a tertiary amine, the following methods are known.
(1) A method for producing an alkadienol, in which a conjugated alkadiene and water are subjected to a telomerization reaction in the presence of a palladium catalyst composed of a palladium compound and an organic phosphorus compound, a tertiary amine, and an organic solvent having high solubility in water, the obtained reaction liquid is distilled to distill the alkadienol and the solvent, a solution containing a palladium catalyst is obtained from a tower bottom liquid, and the solution containing the palladium catalyst is cyclically used for the reaction (see PTLs 1 to 4);
(2) A method for producing 2,7-octadien-1-ol, in which butadiene and water are subjected to a telomerization reaction in the presence of a palladium catalyst composed of a palladium compound and a water-soluble phosphine in an aqueous sulfolane solution containing a carbonate and a bicarbonate of a tertiary amine to produce 2,7-octadien-1-ol, the 2,7-octadien-1-ol is extracted and separated by extracting at least a part of the reacted mixture liquid with a saturated aliphatic hydrocarbon or the like, and at least a part of the sulfolane raffinate containing the palladium catalyst is cyclically used for the reaction (see PTLs 5 to 7);
(3) A method for producing 2,7-octadien-1-ol, in which butadiene and water are subjected to a telomerization reaction in the presence of a tertiary amine having a function as a surfactant in order to compensate for a low reaction rate caused by the low solubility of butadiene in water in a two phase system including a water phase in which a palladium catalyst composed of a palladium compound and a water-soluble phosphorus-containing compound is dissolved in water and butadiene as an organic phase (see PTL 8 and NPL 1); and
(4) A process for producing 2,7-octadien-1-ol, which includes subjecting butadiene and water to a telomerization reaction in the presence of a palladium catalyst composed of a palladium compound and a water-soluble phosphorus-containing compound, a tertiary amine and carbon dioxide, mixing the reaction liquid with a specific organic solvent, and then subjecting the mixture to phase separation in the presence of carbon dioxide (see PTL 9).

### Citation List

### Patent Literature

Patent Literature 1: JP 54-144306 A
Patent Literature 2: JP 5-221897 A
Patent Literature 3: JP 11-189556 A
Patent Literature 4: JP 11-228469 A
Patent Literature 5: JP 64-25739 A
Patent Literature 6: JP 3-232831 A
Patent Literature 7: JP 6-321828 A
Patent Literature 8: JP 8-501800 A
Patent Literature 9: WO 2014/157402

### Non-Patent Literature

Non-Patent Literature 1: Applied Catalysis A: General, Vol. 131, 1995, pp. 167 to 178
Non-Patent Literature 2: Journal of Molecular Catalysis A: Chemical, Vol. 166, 2001, pp. 233 to 242

### Summary of Invention

### Technical Problem

In the production method (1) described in PTLs 1 to 4, when the obtained reaction liquid is distilled, a solution containing a palladium catalyst is recovered from the tower bottom liquid. At that time, in order to suppress metalation of the palladium catalyst, it is necessary to carry out distillation under reduced pressure at a temperature of 110°C or lower. As a result, the palladium catalyst and 2,7-octadien-1-ol inevitably coexist under the condition in which the concentration of carbon dioxide in the system is reduced. When butadiene and a palladium catalyst coexist in the absence of carbon dioxide or under the condition of low carbon dioxide concentration, 1,3,7-octatriene, oligomers, and the like are produced (see NPL 2).

In addition, in the production method (2) described in PTLs 5 to 7, the production method (3) described in PTL 8 and NPL 1, and the production method (4) described in PTL 9, the main emphasis is placed on providing an industrially advantageous method for producing 2,7-octadien-1-ol in which an expensive palladium catalyst is highly efficiently recovered without deteriorating the stability of the palladium catalyst, the palladium catalyst is cyclically reused, and the reaction rate per palladium atom is improved.

As a result of detailed studies on a method for producing 2,7-octadien-1-ol using butadiene and water as raw materials, the present inventors have found that 2,6-octadien-1-ol is produced as a by-product in a non-negligible amount with the progress of the reaction. The production of 2,6-octadien-1-ol is not described or recognized in the above-mentioned prior art documents. 2,6-Octadien-1-ol has a boiling point close to that of 2,7-octadien-1-ol, which makes its separation difficult. This affects the quality of the desired product 2,7-octadien-1-ol. And in some applications, its presence has been found to cause problems.

Therefore, an industrially advantageous method for producing 2,7-octadien-1-ol, which can suppress the production of 2,6-octadien-1-ol, is desired.

An object of the present invention is to provide an industrially advantageous method for producing 2,7-octadien-1-ol, which can improve the productivity of 2,7-octadien-1-ol while suppressing the production of 2,6-octadien-1-ol as a by-product.

### Solution to Problem

As a result of intensive studies to solve the above problems, the present inventor has conceived the following present invention and found that the problems can be solved.

That is, the present invention is as the following [1] to [11].
[1] A method for producing 2,7-octadien-1-ol, including a step (1) of feeding a raw material mixture liquid containing butadiene, a Group 6 to 11 transition metal catalyst containing a Group 6 to 11 transition metal, a tertiary phosphorus compound, an amine compound, and water to a telomerization reactor, and a step (2) of reacting butadiene and water in a carbon dioxide atmosphere in the telomerization reactor to obtain a reaction mixture liquid containing 2,7-octadien-1-ol, in which a residence time of the raw material mixture liquid in the telomerization reactor is 1.3 to 3.0 hours.
[2] The method for producing 2,7-octadien-1-ol according to [1], in which the molar ratio of the amount of 2,6-octadien-1-ol produced to the amount of 2,7-octadien-1-ol produced is 0.01% to 2.0%.
[3] The method for producing 2, 7-octadien-1-ol according to [1] or [2], in which the molar ratio of the total amount of 1,3,7-octatriene and 1,7-octadien-3-ol produced to the amount of 2,7-octadien-1-ol produced is 1.0% to 20.0%.
[4] The method for producing 2,7-octadien-1-ol according to any one of [1] to [3], in which the Group 6 to 11 transition metal catalyst is a palladium compound.
[5] The method for producing 2,7-octadien-1-ol according to [4], in which a palladium concentration in the raw material mixture liquid is 10 to 10,000 ppm by mass.
[6] The method for producing 2,7-octadien-1-ol according to any one of [1] to [5], in which the tertiary phosphorus compound is an aromatic phosphine.
[7] The method for producing 2,7-octadien-1-ol according to [6], in which the aromatic phosphine is a hydrophilic aromatic phosphine.
[8] The method for producing 2,7-octadien-1-ol according to [7], in which the hydrophilic aromatic phosphine is at least one selected from the group consisting of lithium 3-diphenylphosphinobenzenesulfonate, triethylamine 3-diphenylphosphinobenzenesulfonate, and sodium 3-diphenylphosphinobenzenesulfonate.
[9] The method for producing 2,7-octadien-1-ol according to any one of [1] to [8], in which the temperature of the reaction liquid in the telomerization reactor is 50°C to 90°C.
[10] The method for producing 2,7-octadien-1-ol according to any one of [1] to [9], in which the raw material mixture liquid further contains an organic solvent, and the organic solvent is at least one selected from the group consisting of tetrahydrofuran, 1, 4-dioxane, and sulfolane.
[11] The method for producing 2,7-octadien-1-ol according to any one of [1] to [10], in which a content of the tertiary phosphorus compound in the raw material mixture liquid is 0.1 to 100 mol times relative to a content of the Group 6 to 11 transition metal.
[12] The method for producing 2,7-octadien-1-ol according to [1], in which the molar ratio of the amount of 2,6-octadien-1-ol produced to the amount of 2,7-octadien-1-ol produced is 0.01% to 2.0%, the Group 6 to 11 transition metal catalyst is a palladium compound, and a palladium concentration in the raw material mixture liquid is 10 to 10,000 ppm by mass.

### Advantageous Effects of Invention

According to the present invention, it is possible to provide an industrially advantageous method for producing 2, 7-octadien-1-ol, which can improve the productivity of 2,7-octadien-1-ol while suppressing the production of 2,6-octadien-1-ol as a by-product.

### Description of Embodiments

Hereinafter, a description will be given based on an example of an embodiment of the present invention. However, the embodiments shown below are examples for embodying the technical idea of the present invention, and the present invention is not limited to the following description.

In addition, in the description herein, preferred forms of the embodiment are shown, but a combination of two or more of the individual preferred forms is also a preferred form. Regarding the matters indicated by the numerical ranges, in a case where there are several numerical ranges, it is possible to selectively combine the lower limit value and the upper limit value thereof to obtain a preferred embodiment.

In the description herein, when there is a description of a numerical range of "XX to YY", it means "XX or more and YY or less".

### [Method for Producing 2,7-Octadien-1-ol]

The method for producing 2,7-octadien-1-ol of the present invention includes at least a step (1) of feeding a raw material mixture liquid containing butadiene, a Group 6 to 11 transition metal catalyst containing a Group 6 to 11 transition metal, a tertiary phosphorus compound, an amine compound, and water to a telomerization reactor, and a step (2) of reacting butadiene and water in a carbon dioxide atmosphere in the telomerization reactor to obtain a reaction mixture liquid containing 2,7-octadien-1-ol, and if necessary, further includes other steps.

In the method for producing 2,7-octadien-1-ol of the present invention, the residence time of the raw material mixture liquid in the telomerization reactor is 1.3 to 3.0 hours.

The present inventors have found that the amount of 2,6-octadien-1-ol produced can be reduced while increasing the productivity of 2,7-octadien-1-ol by controlling the residence time within the above range.

The "residence time of the raw material mixture liquid" in the description herein is a so-called apparent residence time, and is calculated by "the amount of the reaction mixture liquid in the telomerization reactor ÷ the feed amount of the raw material mixture liquid". In addition, the "amount of the reaction mixture liquid in the telomerization reactor" means the "amount of the liquid remaining in the telomerization reactor".

The amount of the reaction mixture liquid in the telomerization reactor is not particularly limited, and is preferably 0.01 m³ to 1,000 m³, more preferably 0.1 m³ to 500 m³, and still more preferably 1 m³ to 100 m³.

The feed amount of the raw material mixture liquid is not particularly limited, and is preferably 0.003 m³/hour to 769 m³/hour, more preferably 0.03 m³/hour to 385 m³/hour, and still more preferably 0.3 m³/hour to 77 m³/hour.

In the production method of the present invention, butadiene, a Group 6 to 11 transition metal catalyst containing a Group 6 to 11 transition metal, a tertiary phosphorus compound, an amine compound, and water are used as essential components, and if necessary, an organic solvent and other components are used as optional components.

Hereinafter, each component used in the production method of the present invention will be described.

### <Butadiene>

As butadiene, any of industrially available hydrocarbon mixtures usually called C4 fractions in petrochemicals can be used, but since the palladium catalyst is poisoned by acetylene, sulfur, halogen, and the like, it is preferred to use butadiene having a content of these components of 0.1 ppm or less. Such butadiene is called a polymerization grade product or a chemical grade product, and is industrially available.

The content of butadiene in the raw material mixture liquid fed to the telomerization reactor is not particularly limited, and is preferably 1% to 30% by mass, more preferably 3% to 25% by mass, and still more preferably 5% to 20% by mass, from the viewpoint of production efficiency and suppression of a decrease in reaction efficiency associated with two phase separation.

### <Group 6 to 11 Transition Metal Catalyst Containing Group 6 to 11 Transition Metal>

As the Group 6 to 11 transition metal catalyst containing a Group 6 to 11 transition metal used in the production method of the present invention, a compound containing a Group 6 to 11 transition metal in the long periodic table can be used. As the Group 6 to 11 transition metal, one or more selected from the group consisting of Group 6 transition metals and Group 10 transition metals are preferable, Group 10 transition metals are more preferable, and palladium is still more preferable.

More specific examples of the compound containing a Group 6 to 11 transition metal include a molybdenum compound, a tungsten compound, a nickel compound, a palladium compound, and a platinum compound. Among these, a palladium compound is preferable.

Examples of the palladium compound include divalent palladium complexes such as palladium acetate, palladium acetylacetonate, palladium chloride, and palladium nitrate; and 0-valent palladium complexes such as tris(dibenzylideneacetone)dipalladium, tetrakis(triphenylphosphine)palladium, and bis(1,5-cyclooctadiene)palladium. One of these may be used alone, or two or more thereof may be used in combination.

Among these, from the viewpoint of solubility and easy availability, one or more selected from the group consisting of palladium acetate, palladium chloride, and tris (dibenzylideneacetone)dipalladium are preferable, and palladium acetate is more preferable.

The content of the Group 6 to 11 transition metal catalyst in the raw material mixture liquid to be fed to the telomerization reactor is not particularly limited, and is preferably 10 to 1,000 ppm by mass, more preferably 50 to 800 ppm by mass, and still more preferably 100 to 500 ppm by mass from the viewpoint of production efficiency and suppression of the amount of catalyst elution.

The amount of palladium used as a catalyst is not particularly limited, and from the viewpoint of improving the reaction rate, the palladium concentration in the raw material mixture liquid is preferably 10 to 10,000 ppm by mass, more preferably 50 to 500 ppm by mass, and still more preferably 100 to 300 ppm by mass. In addition, from the viewpoint of improving the reaction rate, the amount of palladium is preferably in a range of 0.001 to 100 mmol, and more preferably in a range of 0.01 to 10 mmol in terms of palladium atoms per 1 mol of butadiene. From the viewpoint of suppressing the amount of 1,3,7-octatriene and 1,7-octadien-3-ol produced, the palladium concentration in the raw material mixture is preferably 450 to 650 ppm by mass, and more preferably 500 to 600 ppm by mass.

### <Tertiary Phosphorus Compound>

In the production method of the present invention, a tertiary phosphorus compound as a ligand capable of forming a complex by coordinating to a Group 6 to 11 transition metal is used. As the tertiary phosphorus compound, phosphine, phosphite, phosphonite, or the like is preferable.

Specific examples of the tertiary phosphorus compound include aliphatic phosphines such as triisopropylphosphine, tri-n-butylphosphine, and tri-n-octylphosphine; alicyclic phosphines such as tricyclohexylphosphine; hydrophobic aromatic phosphines such as triphenylphosphine, tritolylphosphine, diphenyl-p-chlorophenylphosphine, and trimesitylphosphine; hydrophilic aromatic phosphines such as lithium 3-diphenylphosphinobenzenesulfonate, sodium 3-diphenylphosphinobenzenesulfonate, triethylamine 3-diphenylphosphinobenzenesulfonate, and tris(sodium 3-sulfophenyl)phosphine; phosphites such as triethyl phosphite, tributyl phosphite, and triisopropyl phosphite; and phosphonites such as octyldioctoxyphosphine and butyldibutoxyphosphine. One of these may be used alone, or two or more thereof may be used in combination.

Among these, from the viewpoint of stability in the reaction system, an aromatic phosphine, that is, at least one of a hydrophobic aromatic phosphine and a hydrophilic aromatic phosphine is preferable, a hydrophilic aromatic phosphine is more preferable, and at least one of lithium 3-diphenylphosphinobenzenesulfonate, triethylamine 3-diphenylphosphinobenzenesulfonate, and sodium 3-diphenylphosphinobenzenesulfonate is particularly preferable.

The hydrophilic aromatic phosphine refers to an aromatic phosphine having a hydrophilic group, such as a group represented by -SO₃M, -COOM (M represents an alkali metal ion or H⁺N(R¹¹)(R¹²)(R¹³)), or N(R¹⁴)(R¹⁵). Here, R¹¹, R¹², and R¹³ each independently represent a hydrogen atom or an alkyl group having 1 to 3 carbon atoms, and R¹⁴ and R¹⁵ each independently represent an alkyl group having 1 to 3 carbon atoms.

The hydrophobic aromatic phosphine refers to an aromatic phosphine having no hydrophilic group.

The amount of the tertiary phosphorus compound present in the raw material mixture liquid is not particularly limited, and can be determined in consideration of the ratio to the Group 6 to 11 transition metal contained in the Group 6 to 11 transition metal catalyst, preferably palladium atom. From the viewpoint of the stability of the catalyst, the rate of the telomerization reaction, the cost, and the like, the content of the tertiary phosphorus compound in the raw material mixture liquid is preferably 0.1 to 100 mol times, more preferably 1 to 50 mol times, and still more preferably 5 to 30 mol times, with respect to the content of the Group 6 to 11 transition metal.

Further, when a tertiary phosphorus compound, preferably a hydrophilic aromatic phosphine, is additionally introduced in the telomerization reaction step (step (2)), it is preferable to adjust the amount of the tertiary phosphorus compound to be within this range. When the amount is 0.1 mol times or more, the formation of catalytically active species becomes sufficient, it is possible to suppress side reactions to vinylcyclohexene and the like, and thus to suppress a decrease in the yield of 2,7-octadien-1-ol, and it is also possible to suppress a decrease in the recovery rate of a Group 6 to 11 transition metal catalyst containing a Group 6 to 11 transition metal, preferably a palladium catalyst. On the other hand, when the amount is 100 mol times or less, the coordination of butadiene to the palladium catalyst is not inhibited, and the reaction rate can be maintained.

When a hydrophilic aromatic phosphine is used as the tertiary phosphorus compound in the telomerization reaction, the hydrophilic aromatic phosphine coexists with carbon dioxide, water, and a tertiary amine. Therefore, as described in JP 2003-171388 A and the like, M in the sulfonic acid group (-SO₃M) contained in the hydrophilic aromatic phosphine may be an ammonium cation derived from the tertiary amine used in the reaction. Further, the sulfonic acid group (-SO₃M) may be ionized into -SO₃⁻ and M⁺.

In the telomerization reaction, the tertiary phosphorus compound coexists with an alkenyl compound such as 2,7-octadien-1-ol which is a product. Therefore, as described in JP 2002-371089 A and the like, the tertiary phosphorus compound (preferably hydrophilic aromatic phosphine) may form a phosphonium salt by reaction with 2,7-octadien-1-ol or the like.

The content of the tertiary phosphorus compound in the raw material mixture liquid to be fed to the telomerization reactor is not particularly limited, and is preferably 0.1% to 30% by mass, more preferably 0.5% to 20% by mass, and still more preferably 1% to 10% by mass from the viewpoint of stabilization of the transition metal catalyst and suppression of precipitation of the tertiary phosphorus compound.

### <Amine Compound>

In the production method of the present invention, the amine compound can react with water and carbon dioxide in the reaction system to produce an ammonium ion and a hydrogen carbonate ion. The hydrogen carbonate ion can produce 2,7-octadien-1-ol by attacking a reaction intermediate formed from one atom of a Group 6 to 11 transition metal contained in the Group 6 to 11 transition metal catalyst, preferably palladium, one molecule or two molecules of a tertiary phosphorus compound, two molecules of butadiene, and the like.

The amine compound is not particularly limited, and may be an ammonium salt, but is preferably a tertiary amine, more preferably a monodentate tertiary amine having a basicity constant (pKa) of 7 or more, and examples thereof include trimethylamine, triethylamine, tri-n-butylamine, 1-N,N-dimethylamino-2-propanol, N,N-dimethyl-2-methoxyethylamine, N-methylmorpholine, and N,N,N',N'-tetramethylhexamethylenediamine. Among these, trimethylamine and triethylamine are preferable in consideration of various points such as reaction performance, boiling point, solubility, and price.

The mass ratio of the amine compound to water [amine compound/water] is preferably 0.1 to 10, and more preferably 0.5 to 5. When this mass ratio is 0.1 or more, the hydrogen carbonate ion concentration in the reaction system becomes sufficiently high, so that the reaction rate is also increased, and at the same time, the by-production of 1,3,7-octatriene and vinylcyclohexene can be suppressed. When this mass ratio is 10.0 or less, the coordination of the tertiary phosphorus compound to palladium is not likely to be inhibited, and as a result, the by-production of 1,3,7-octatriene and vinylcyclohexene can be suppressed.

The content of the amine compound in the raw material mixture liquid to be fed to the telomerization reactor is not particularly limited, and is preferably 0.1% to 20% by mass, more preferably 1% to 15% by mass, and still more preferably 3% to 10% by mass, from the viewpoint of improving the reaction rate and ensuring the volume efficiency.

The monodentate tertiary amine reacts with water and carbon dioxide in the reaction system and exists as an equilibrium mixture (carbonate and/or hydrogen carbonate) of (i) ammonium ions and (ii) carbonate ions and hydrogen carbonate ions, and the existing ratio of the carbonate and/or hydrogen carbonate of the amine compound under the reaction conditions depends on the reaction liquid temperature and the partial pressure of carbon dioxide. From the viewpoint of further improving the productivity of 2,7-octadien-1-ol, it is preferable to determine the amount of carbon dioxide using pressure or the like as an index.

### <Water>

As the water, water having a purity that does not adversely affect the telomerization reaction is preferably used. For example, industrially, it is preferable to use ion-exchanged water or drain water obtained by condensing steam. When iron rust or the like is contained, the tertiary phosphorus compound forms a complex with a metal ion derived therefrom, so that the formation of the palladium catalyst becomes insufficient, and a side reaction to vinylcyclohexene or the like occurs, so that the yield of 2,7-octadien-1-ol decreases and the recovery rate of the palladium catalyst decreases.

The content of water in the raw material mixture liquid to be fed to the telomerization reactor is not particularly limited, and is preferably 1% to 50% by mass, more preferably 5% to 40% by mass, and still more preferably 10% to 30% by mass, from the viewpoint of suppressing a decrease in the reaction rate and suppressing a decrease in the reaction efficiency due to two phase separation.

### <Organic Solvent>

In the production method of the present invention, the organic solvent used as an optional component as necessary is not particularly limited, and it is preferable that the organic solvent can be used in the presence of an organic solvent that can at least partially dissolve butadiene, a Group 6 to 11 transition metal catalyst containing a Group 6 to 11 transition metal, a tertiary phosphorus compound, and an amine compound and does not adversely affect the telomerization reaction. Examples of such solvents include ethers such as diethyl ether, tetrahydrofuran, and 1,4-dioxane; ketones such as acetone and methyl ethyl ketone; nitriles such as acetonitrile and benzonitrile; sulfones such as sulfolane and methylsulfolane; esters such as methyl acetate and ethyl acetate; aromatic hydrocarbons such as benzene, toluene, and xylene; and aliphatic hydrocarbons such as butane, hexane, and cyclohexane. One of these may be used alone, or two or more thereof may be used in combination.

Among these, at least one selected from the group consisting of tetrahydrofuran, 1,4-dioxane, and sulfolane is preferable, and sulfolane is more preferable. Sulfolane is preferred because it is miscible with both butadiene and water, allows the separation of 2,7-octadien-1-ol by extraction from the reaction mixture liquid, and does not adversely affect the reaction even in long-term continuous use.

The mass ratio of water to sulfolane in the reaction mixture liquid is preferably in the range of water/sulfolane = 10/90 to 70/30, more preferably in the range of 20/80 to 40/60, from the viewpoint of improving the reaction rate and efficiently performing the extraction of 2,7-octadien-1-ol.

The content of the organic solvent in the raw material mixture liquid to be fed to the telomerization reactor is not particularly limited, and is preferably 1% to 80% by mass, more preferably 20% to 70% by mass, and still more preferably 30% to 60% by mass, from the viewpoint of ensuring the volume efficiency and suppressing a decrease in the reaction efficiency due to two phase separation.

### <Telomerization Reaction Step>

A raw material mixture liquid containing butadiene, a Group 6 to 11 transition metal catalyst containing a Group 6 to 11 transition metal, a tertiary phosphorus compound, an amine compound, and water is fed to a telomerization reactor (step (1)) and subjected to a telomerization reaction. That is, butadiene and water are reacted under a carbon dioxide atmosphere in a telomerization reactor in the presence of a Group 6 to 11 transition metal catalyst containing a Group 6 to 11 transition metal (preferably palladium) and a tertiary phosphorus compound (preferably a hydrophilic aromatic phosphine) (in a state where optional components such as an organic solvent are further present as necessary) to obtain a reaction mixture liquid containing 2,7-octadien-1-ol (step (2)).

Here, "the raw material mixture liquid is fed to the telomerization reactor" includes not only a case where the raw material mixture liquid is prepared by mixing the raw materials in advance and then the raw material mixture liquid is fed to the telomerization reactor, but also a case where a part or all of the raw materials are fed to the telomerization reactor without being mixed in advance, and the former is preferable.

The reaction can preferably be carried out using a complete mixing reactor. The reaction can be carried out in a batch system (including a semi-continuous system) or a continuous flow system, and can also be carried out in a continuous flow system by connecting two or three complete mixing reactors in series. From the viewpoint of obtaining an industrially satisfactory level of productivity of 2,7-octadien-1-ol, the reaction is preferably carried out in a continuous flow system. That is, the step (1) and the step (2) in the production method of the present invention are preferably carried out not sequentially but simultaneously.

The term "complete mixing reactor" as used herein refers to a reactor designed so that raw materials supplied into the reactor are mixed into a substantially uniformly dispersed state without a moment of time.

In a continuous flow system using a complete mixing reactor as the telomerization reactor, the mass ratio of butadiene to 2,7-octadien-1-ol present in the telomerization reactor in a steady state can be appropriately set according to a desired butadiene conversion rate. From the viewpoint of achieving the productivity of 2,7-octadien-1-ol at an industrially satisfactory level, the mass ratio of butadiene and 2,7-octadien-1-ol to water [(butadiene + 2,7-octadien-1-ol)/water] present in the reaction system is preferably 0.1 to 50, and more preferably 0.5 to 10.

Any carbon dioxide may be used as long as it is present as carbon dioxide in the reaction system, and for example, it can be supplied as molecular carbon dioxide, carbonic acid, a carbonate, or a bicarbonate, and it is preferable to use molecular carbon dioxide and perform the reaction under carbon dioxide pressure in order to increase the solubility of molecular carbon dioxide in the reaction system. The partial pressure of carbon dioxide is in a state of 0.05 MPa to 5 MPa (absolute pressure).

In addition, it is preferable that the reaction pressure of the entire reaction system and the vapor pressures of butadiene, the reaction product, and the solvent under the reaction temperature (reaction liquid temperature) are also in the range of 0.101 MPa (normal pressure) to 9.8 MPa. Further, a gas inert to the reaction, such as nitrogen or argon, may be allowed to coexist.

The telomerization reaction is preferably performed at a temperature (reaction liquid temperature) of 50°C to 90°C. From the viewpoint of further improving the productivity of 2,7-octadien-1-ol, the telomerization reaction is more preferably performed at a temperature of 50°C to 80°C, and still more preferably performed at a temperature of 65°C to 75°C. That is, the temperature in the telomerization reactor is preferably 50°C to 90°C, more preferably 50°C to 80°C, and still more preferably 65°C to 75°C.

The method of the present invention is characterized in that the residence time of the raw material mixture liquid in the telomerization reactor is controlled to 1.3 to 3.0 hours. The residence time may also vary depending on the temperature of the reaction liquid, the amount of the Group 6 to 11 transition metal catalyst containing the Group 6 to 11 transition metal (preferably palladium) used as a catalyst, the amount of the tertiary phosphorus compound (preferably hydrophilic aromatic phosphine) used, the supply amount of butadiene, the amount of the amine compound used, the target conversion rate of butadiene, and the like, and is preferably in the range of 1.3 to 2.5 hours and more preferably 1.5 to 2.4 hours.

By controlling the residence time within the above range, the productivity of 2,7-octadien-1-ol can be increased and at the same time, the amount of 2,6-octadien-1-ol produced can be reduced.

Examples of the reaction product of the telomerization reaction in which butadiene and water are reacted in a carbon dioxide atmosphere include, in addition to 2,7-octadien-1-ol which is the target product, 1,7-octadien-3-ol, 1,3,7-octatriene, vinylcyclohexene, and 2,6-octadien-1-ol which has been found to be produced in a non-negligible amount.

According to the production method of the present invention, the molar ratio of the amount of 2,6-octadien-1-ol produced to the amount of 2,7-octadien-1-ol produced is preferably controlled in the range of 0.01% to 2.0%, more preferably controlled in the range of 0.01% to 1.5%, and still more preferably controlled in the range of 0.01% to 1.0%.

In addition, in the production method of the present invention, the molar ratio of the total of the amounts of 1,3,7-octatriene and 1,7-octadien-3-ol produced to the amount of 2,7-octadien-1-ol produced is preferably controlled in the range of 1.0% to 20.0%, more preferably controlled in the range of 1.0% to 15.0%, and still more preferably controlled in the range of 1.0% to 10.0%.

After completion of the telomerization reaction, a catalyst component composed of a tertiary phosphorus compound and a palladium compound is separated and recovered from the reaction mixture liquid by a distillation method, an extraction method, or the like.

It is desirable to recover the catalyst component by an extraction method which is less likely to deteriorate the activity of the catalyst component and can recycle the catalyst component over a longer period of time. The extraction method is carried out, for example, by carrying out the telomerization reaction and then subjecting the reaction mixture liquid to an extraction operation using a solvent immiscible with the reaction solvent as an extractant. In the method of the present invention, by using a hydrophilic aromatic phosphine as the tertiary phosphorus compound and preferably using an organic solvent miscible with water such as sulfolane, the catalyst component can be separated as a raffinate component into a phase containing water and sulfolane. By returning the separated catalyst component to the reaction system as it is, the loss of the catalyst component can be suppressed and the catalyst component can be cyclically used.

The organic phase obtained by the extraction operation contains butadiene, 2,7-octadien-1-ol, other by-products, amine compounds, and optionally carbon dioxide. Carbon dioxide and butadiene are removed by flashing the organic phase, and if necessary, at least a part of the removed carbon dioxide and butadiene is again fed to the telomerization reactor as a component of the raw material mixture liquid and cyclically reused.

The amine compound, 2,7-octadien-1-ol, other by-products, and the like can be separated by distillation. The separated amine compound may be reused in the telomerization reaction.

In the production method of the present invention, 2,7-octadien-1-ol may be separated by extracting at least a part of the reaction mixture liquid with an extractant.

The extractant that can be used is not particularly limited, and examples thereof include a saturated aliphatic hydrocarbon, a monoolefinic hydrocarbon, and an alicyclic hydrocarbon, which have a boiling point lower than that of 2,7-octadien-1-ol.

Specific examples of the extractant include saturated aliphatic hydrocarbons such as n-butane, isobutane, n-pentane, n-hexane, n-heptane, n-octane, and isooctane; monoolefinic hydrocarbons such as butene and isooctane; and alicyclic hydrocarbons such as cyclohexane, cyclohexene, and methylcyclohexane. One of these may be used alone, or two or more thereof may be used in combination.

Among these, n-hexane is preferable.

Here, a mixture of hydrocarbons such as butane, butene, and isobutene contained in the C₄ fraction as the butadiene source can also be used.

In consideration of the extraction efficiency of 2,7-octadien-1-ol and the like, the extractant is preferably used in an amount within a range of 0.3 to 3.0 in terms of a volume ratio with respect to the reaction mixture liquid.

The raffinate layer (aqueous solution layer) containing the catalyst component is cyclically reused in the synthesis of 2,7-octadien-1-ol. If desired, a part of the raffinate layer may be taken out, subjected to a catalyst activation treatment, and then recycled to the above synthesis.

As an extraction apparatus used in the extraction, an industrially general-purpose stirring type extraction tower, an RDC type extraction tower, a perforated plate tower, or the like can be applied. Industrially, the extraction operation is carried out in a continuous manner by providing a standing tank sufficient for phase separation. The 2,7-octadien-1-ol is optionally purified from the extraction layer by conventional separation means such as distillation.

The fraction containing 2,7-octadien-1-ol, which is the target product, as a main component can be purified by distillation under reduced pressure. The distillation temperature of the fraction containing 2,7-octadien-1-ol as a main component can be appropriately selected depending on the pressure in the distillation system, and is preferably 200°C or lower, and more preferably 140°C or lower, because the reaction of 2,7-octadien-1-ol to 1,3,7-octatriene or the like proceeds when the fraction is heated to around 200°C in the presence of a trace amount of a palladium catalyst.

### Examples

Hereinafter, the present invention will be described in more detail with reference to Examples, but the present invention is not limited to these Examples.

Unless otherwise specified, operations were performed under a nitrogen atmosphere.

### <Measurement and Evaluation Method>

Quantitative determination was performed by performing gas chromatography analysis on the reaction mixture liquid after the telomerization reaction under the following measurement conditions. The results are shown in Table 1.

### (Gas Chromatography Analysis Conditions)

Apparatus: GC-2014 (manufactured by Shimadzu Corporation)
Column used: DB-1701 (inside diameter 0.32 mm × length 50 m, film thickness 1 µm) manufactured by Agilent Technologies Inc.
Analysis conditions: inlet temperature 250°C, detector temperature 250°C
Sample injection volume: 0.2 µL
Carrier gas: helium (80 kPa) flowing at 30 cm/s
Column temperature: Keep at 50°C for 35 minutes → Raise the temperature at 10°C/min → Keep at 220°C for 20 minutes
Detector: flame ionization detector (FID)

The conversion rate of butadiene was calculated by Equation 1 below. The unit of each amount in the equation is mol. The calculation results are shown in Table 1. The "amount of butadiene in the reaction liquid" in Equation 1 means the "amount of butadiene in the reaction mixture liquid", and the "amount of charged butadiene" means the "amount of butadiene in the raw material mixture liquid".Butadiene conversion rate (%) = 100 × [1 - (Amount of butadiene in the reaction liquid)/(Amount of charged butadiene)]

Examples of the respective products include 2,7-octadien-1-ol (2,7-ODA), 2,6-octadien-1-ol (2,6-ODA), 1,3,7-octatriene (OCT), and 1,7-octadien-3-ol (IODA). The selectivity (%) of each product was calculated by Equation 2 below. The unit of each amount in the equation is mol. The calculation results are shown in Table 1. In Equation 2, "reaction liquid" means "reaction mixture liquid".Selectivity of each product (%) = 200 × (Amount of each product in the reaction liquid)/(Amount of reacted butadiene)

### [OCT + IODA]

The sum of the above-calculated "selectivity (mol%) of 1,3,7-octatriene (OCT)" and the above-calculated "selectivity (mol%) of 1,7-octadien-3-ol (IODA)" was calculated, and the calculated sum is shown in the column of "OCT + IODA" in Table 1.

### [2,7-ODA/(2,7-ODA + IODA)]

The ratio of the above-calculated "selectivity (mol%) of 2,7-octadien-1-ol (2,7-ODA)" to the sum of the above-calculated "selectivity (mol%) of 1,7-octadien-3-ol (IODA)" and the above-calculated "selectivity (mol%) of 2,7-octadien-1-ol (2,7-ODA)" was calculated, and the calculated ratio is shown in the column of "2,7-ODA/(2,7-ODA + IODA)" in Table 1.

### [2,7-ODA/(2,7-ODA + 2,6-ODA)]

The ratio of the above-calculated "selectivity (mol%) of 2,7-octadien-1-ol (2,7-ODA)" to the sum of the above-calculated "selectivity (mol%) of 2,6-octadien-1-ol (2,6-ODA)" and the above-calculated "selectivity (mol%) of 2,7-octadien-1-ol (2,7-ODA)" was calculated, and the calculated ratio is shown in the column of "2,7-ODA/(2,7-ODA + 2,6-ODA)" in Table 1.

### <Examples 1 to 5 and Comparative Examples 1 and 2>

### (Example 1)

To a telomerization reactor at a reaction liquid temperature of 74°C, a raw material mixture liquid having a component composition containing 11.2% by mass of butadiene (Bd) as a monomer, 4.8% by mass of triethylamine, 21.1% by mass of water, 9.8% by mass of 2,7-octadien-1-ol (2,7-ODA), palladium (derived from 410 ppm by mass of palladium acetate (Pd(OAc)₂)) as a catalyst, triethylamine 3-diphenylphosphinobenzenesulfonate (tertiary phosphorus compound) of 20 mol times (1.7% by mass) relative to palladium (Pd), and sulfolane (SLF) as a solvent of the remaining mass ratio (51.4% by mass) when the total was 100% by mass was fed (step (1)). Through the above step (1), the reaction mixture liquid became a steady state.

Butadiene and water were reacted in a carbon dioxide atmosphere in the telomerization reactor, and a reaction mixture liquid containing 2,7-octadien-1-ol was obtained by setting the residence time of the raw material mixture liquid in the telomerization reactor to 1.3 hours (step (2)). The butadiene conversion rate was 59 mol%, and the component composition of the product in the reaction mixture liquid was 88 mol% of 2,7-octadien-1-ol (2,7-ODA), 0.4 mol% of 2,6-octadien-1-ol (2,6-ODA), 2.3 mol% of 1,3,7-octatriene (OCT), and 5.0 mol% of 1,7-octadien-3-ol (IODA). The total pressure was adjusted to 1.4 MPaG with carbon dioxide.

### (Example 2)

To a telomerization reactor at a reaction liquid temperature of 74°C, a raw material mixture liquid having a component composition containing 13.8% by mass of butadiene (Bd) as a monomer, 3.0% by mass of triethylamine, 20.5% by mass of water, 10.1% by mass of 2,7-octadien-1-ol (2,7-ODA), palladium (derived from 300 ppm by mass of palladium acetate (Pd(OAc)₂)) as a catalyst, triethylamine 3-diphenylphosphinobenzenesulfonate (tertiary phosphorus compound) of 20 mol times (1.2% by mass) relative to palladium (Pd), and sulfolane (SLF) as a solvent of the remaining mass ratio (51.4% by mass) when the total was 100% by mass was fed (step (1)). Through the above step (1), the reaction mixture liquid became a steady state.

Butadiene and water were reacted in a carbon dioxide atmosphere in the telomerization reactor, and a reaction mixture liquid containing 2,7-octadien-1-ol was obtained by setting the residence time of the raw material mixture liquid in the telomerization reactor to 2.1 hours (step (2)). The butadiene conversion rate was 51 mol%, and the component composition of the product in the reaction mixture liquid was 90 mol% of 2,7-octadien-1-ol (2,7-ODA), 0.5 mol% of 2,6-octadien-1-ol (2,6-ODA), 3.1 mol% of 1,3,7-octatriene (OCT), and 4.1 mol% of 1,7-octadien-3-ol (IODA). The total pressure was adjusted to 1.4 MPaG with carbon dioxide.

### (Example 3)

To a telomerization reactor at a reaction liquid temperature of 72°C, a raw material mixture liquid having a component composition containing 6.6% by mass of butadiene (Bd) as a monomer, 3.7% by mass of triethylamine, 23.6% by mass of water, 12.0% by mass of 2,7-octadien-1-ol (2,7-ODA), palladium (derived from 420 ppm by mass of palladium acetate (Pd(OAc)₂)) as a catalyst, triethylamine 3-diphenylphosphinobenzenesulfonate (tertiary phosphorus compound) of 20 mol times (1.7% by mass) relative to palladium (Pd), and sulfolane (SLF) as a solvent of the remaining mass ratio (52.4% by mass) when the total was 100% by mass was fed (step (1)). Through the above step (1), the reaction mixture liquid became a steady state.

Butadiene and water were reacted in a carbon dioxide atmosphere in the telomerization reactor, and a reaction mixture liquid containing 2,7-octadien-1-ol was obtained by setting the residence time of the raw material mixture liquid in the telomerization reactor to 1.5 hours (step (2)). The butadiene conversion rate was 52 mol%, and the component composition of the product in the reaction mixture liquid was 89 mol% of 2,7-octadien-1-ol (2,7-ODA), 0.5 mol% of 2,6-octadien-1-ol (2,6-ODA), 2.3 mol% of 1,3,7-octatriene (OCT), and 4.5 mol% of 1,7-octadien-3-ol (IODA). The total pressure was adjusted to 1.4 MPaG with carbon dioxide.

### (Example 4)

To a telomerization reactor at a reaction liquid temperature of 66°C, a raw material mixture liquid having a component composition containing 6.6% by mass of butadiene (Bd) as a monomer, 3.7% by mass of triethylamine, 22.9% by mass of water, 12.3% by mass of 2,7-octadien-1-ol (2,7-ODA), palladium (derived from 560 ppm by mass of palladium acetate (Pd(OAc)₂)) as a catalyst, triethylamine 3-diphenylphosphinobenzenesulfonate (tertiary phosphorus compound) of 20 mol times (2.3% by mass) relative to palladium (Pd), and sulfolane (SLF) as a solvent of the remaining mass ratio (52.2% by mass) when the total was 100% by mass was fed (step (1)). Through the above step (1), the reaction mixture liquid became a steady state.

Butadiene and water were reacted in a carbon dioxide atmosphere in the telomerization reactor, and a reaction mixture liquid containing 2,7-octadien-1-ol was obtained by setting the residence time of the raw material mixture liquid in the telomerization reactor to 1.5 hours (step (2)). The butadiene conversion rate was 50 mol%, and the component composition of the product in the reaction mixture liquid was 91 mol% of 2,7-octadien-1-ol (2,7-ODA), 0.6 mol% of 2,6-octadien-1-ol (2,6-ODA), 1.9 mol% of 1,3,7-octatriene (OCT), and 3.8 mol% of 1,7-octadien-3-ol (IODA). The total pressure was adjusted to 1.4 MPaG with carbon dioxide.

### (Example 5)

To a telomerization reactor at a reaction liquid temperature of 72°C, a raw material mixture liquid having a component composition containing 6.6% by mass of butadiene (Bd) as a monomer, 3.7% by mass of triethylamine, 23.5% by mass of water, 12.9% by mass of 2,7-octadien-1-ol (2,7-ODA), palladium (derived from 280 ppm by mass of palladium acetate (Pd(OAc)₂)) as a catalyst, triethylamine 3-diphenylphosphinobenzenesulfonate (tertiary phosphorus compound) of 20 mol times (1.2% by mass) relative to palladium (Pd), and sulfolane (SLF) as a solvent of the remaining mass ratio (52.1% by mass) when the total was 100% by mass was fed (step (1)). Through the above step (1), the reaction mixture liquid became a steady state.

Butadiene and water were reacted in a carbon dioxide atmosphere in the telomerization reactor, and a reaction mixture liquid containing 2,7-octadien-1-ol was obtained by setting the residence time of the raw material mixture liquid in the telomerization reactor to 2.4 hours (step (2)). The butadiene conversion rate was 53 mol%, and the component composition of the product in the reaction mixture liquid was 91 mol% of 2,7-octadien-1-ol (2,7-ODA), 0.6 mol% of 2,6-octadien-1-ol (2,6-ODA), 2.4 mol% of 1,3,7-octatriene (OCT), and 4.1 mol% of 1,7-octadien-3-ol (IODA). The total pressure was adjusted to 1.4 MPaG with carbon dioxide.

### (Comparative Example 1)

To a telomerization reactor at a reaction liquid temperature of 64°C, a raw material mixture liquid having a component composition containing 6.6% by mass of butadiene (Bd) as a monomer, 3.7% by mass of triethylamine, 23.5% by mass of water, 12.9% by mass of 2,7-octadien-1-ol (2,7-ODA), palladium (derived from 280 ppm by mass of palladium acetate (Pd(OAc)₂)) as a catalyst, triethylamine 3-diphenylphosphinobenzenesulfonate (tertiary phosphorus compound) of 20 mol times (1.2% by mass) relative to palladium (Pd), and sulfolane (SLF) as a solvent of the remaining mass ratio (52.1% by mass) when the total was 100% by mass was fed (step (1)). Through the above step (1), the reaction mixture liquid became a steady state.

Butadiene and water were reacted in a carbon dioxide atmosphere in the telomerization reactor, and a reaction mixture liquid containing 2,7-octadien-1-ol was obtained by setting the residence time of the raw material mixture liquid in the telomerization reactor to 4.0 hours (step (2)). The butadiene conversion rate was 54 mol%, and the component composition of the product in the reaction mixture liquid was 87 mol% of 2,7-octadien-1-ol (2,7-ODA), 2.0 mol% of 2,6-octadien-1-ol (2,6-ODA), 2.2 mol% of 1,3,7-octatriene (OCT), and 4.2 mol% of 1,7-octadien-3-ol (IODA). The total pressure was adjusted to 1.4 MPaG with carbon dioxide.

### (Comparative Example 2)

To a telomerization reactor at a reaction liquid temperature of 74°C, a raw material mixture liquid having a component composition containing 6.6% by mass of butadiene (Bd) as a monomer, 3.7% by mass of triethylamine, 22.9% by mass of water, 12.3% by mass of 2,7-octadien-1-ol (2,7-ODA), palladium (derived from 560 ppm by mass of palladium acetate (Pd(OAc)₂)) as a catalyst, triethylamine 3-diphenylphosphinobenzenesulfonate (tertiary phosphorus compound) of 20 mol times (2.3% by mass) relative to palladium (Pd), and sulfolane (SLF) as a solvent of the remaining mass ratio (52.2% by mass) when the total was 100% by mass was fed (step (1)). Through the above step (1), the reaction mixture liquid became a steady state.

Butadiene and water were reacted in a carbon dioxide atmosphere in the telomerization reactor, and a reaction mixture liquid containing 2,7-octadien-1-ol was obtained by setting the residence time of the raw material mixture liquid in the telomerization reactor to 1.2 hours (step (2)). The butadiene conversion rate was 55 mol%, and the component composition of the product in the reaction mixture liquid was 86 mol% of 2,7-octadien-1-ol (2,7-ODA), 0.5 mol% of 2,6-octadien-1-ol (2,6-ODA), 4.5 mol% of 1,3,7-octatriene (OCT), and 4.1 mol% of 1,7-octadien-3-ol (IODA). The total pressure was adjusted to 1.4 MPaG with carbon dioxide.

### [Table 1]

**Table 1**

| | | | Example | | | | | Comparative Example | |
|---|---|---|---|---|---|---|---|---|---|
| | | | 1 | 2 | 3 | 4 | 5 | 1 | 2 |
| Reaction liquid temperature | | °C | 74 | 74 | 72 | 66 | 72 | 64 | 74 |
| Residence time | | h | 1.3 | 2.1 | 1.5 | 1.5 | 2.4 | 4.0 | 1.2 |
| Content of each component in raw material mixture | Triethylamine | % by mass | 4.8 | 3.0 | 3.7 | 3.7 | 3.7 | 3.7 | 3.7 |
| | Pd(OAc)2 | ppm by mass | 410 | 300 | 420 | 560 | 280 | 280 | 560 |
| | Bd | % by mass | 11.2 | 13.8 | 6.6 | 6.6 | 6.6 | 6.6 | 6.6 |
| | 2,7-ODA | % by mass | 9.8 | 10.1 | 12.0 | 12.3 | 12.9 | 12.9 | 12.3 |
| | Water | % by mass | 21.1 | 20.5 | 23.6 | 22.9 | 23.5 | 23.5 | 22.9 |
| | Tertiary phosphorus compound | % by mass | 1.7 | 1.2 | 1.7 | 2.3 | 1.2 | 1.2 | 2.3 |
| | | Molar ratio to Pd (tertiary phosphorus compound/Pd) (times) | 20 | 20 | 20 | 20 | 20 | 20 | 20 |
| | SLF | % by mass | 51.4 | 51.4 | 52.4 | 52.2 | 52.1 | 52.1 | 52.2 |
| Conversion rate | | mol% | 59 | 51 | 52 | 50 | 53 | 54 | 55 |
| Component composition of product in reaction mixture | 2,7-ODA | mol% | 88 | 90 | 89 | 91 | 91 | 87 | 86 |
| | 2,6-ODA | mol% | 0.4 | 0.5 | 0.5 | 0.6 | 0.6 | 2.0 | 0.5 |
| | OCT | mol% | 2.3 | 3.1 | 2.3 | 1.9 | 2.4 | 2.2 | 4.5 |
| | IODA | mol% | 5.0 | 4.1 | 4.5 | 3.8 | 4.1 | 4.2 | 4.1 |
| | OCT + IODA | mol% | 7.3 | 7.2 | 6.8 | 5.7 | 6.5 | 6.4 | 8.6 |
| 2,7-ODA/(2,7-ODA + IODA) | | | 94.6 | 95.6 | 95.2 | 96.0 | 95.7 | 95.4 | 95.4 |
| 2,7-ODA/(2,7-ODA + 2,6-ODA) | | | 99.5 | 99.4 | 99.4 | 99.3 | 99.3 | 97.8 | 99.4 |

From Table 1, it is found that Examples 1 to 5 in which the residence time of the raw material mixture liquid in the telomerization reactor is within the range of 1.3 to 3.0 hours can improve the productivity of 2,7-octadien-1-ol while suppressing the production of 2,6-octadien-1-ol as a by-product, as compared to Comparative Examples 1 and 2 in which the residence time of the raw material mixture liquid in the telomerization reactor is outside the range of 1.3 to 3.0 hours.

From Table 1, it is found that in Examples 1 to 5 in which the butadiene conversion rates are almost the same, since the "content of butadiene (Bd) in the reaction mixture" is larger in Examples 1 and 2 than in Examples 3 to 5 (Examples 1 and 2: 11.2% to 13.8% by mass, Examples 3 to 5: 6.6% by mass), the production amount of 2,7-octadien-1-ol (2,7-ODA) is larger.

From the comparison between Example 4 and Comparative Example 2 in Table 1, which have the same conditions except for the reaction liquid temperature and the residence time, it is found that in Example 4, the production of the by-product (OCT + IODA) can be reduced, and the productivity of 2,7-octadien-1-ol (2,7-ODA) is improved.

### Industrial Applicability

The 2,7-octadien-1-ol obtained by the production method of the present invention can be derived into 7-octenal by isomerization using a copper-based catalyst, and thus is useful as a raw material of 7-octenal. 7-Octenal is a compound having a highly reactive terminal double bond and an aldehyde group, and is useful as a raw material for various industrial chemicals. Specifically, 1,9-nonanedial is produced by subjecting 7-octenal to a hydroformylation reaction, and 1,9-nonanediamine useful as a raw material for a polymer monomer can be produced by further subjecting the 1,9-nonanedial to a reductive amination reaction.

In addition, the 2,7-octadien-1-ol obtained by the production method of the present invention has an extremely low content of 2,6-octadien-1-ol, and therefore is also useful as a raw material for pharmaceuticals, agricultural chemicals, and the like.

## Claims

1. A method for producing 2,7-octadien-1-ol, comprising a step (1) of feeding a raw material mixture liquid containing butadiene, a Group 6 to 11 transition metal catalyst containing a Group 6 to 11 transition metal, a tertiary phosphorus compound, an amine compound, and water to a telomerization reactor, and a step (2) of reacting butadiene and water in a carbon dioxide atmosphere in the telomerization reactor to obtain a reaction mixture liquid containing 2,7-octadien-1-ol, wherein a residence time of the raw material mixture liquid in the telomerization reactor is 1.3 to 3.0 hours.

2. The method for producing 2,7-octadien-1-ol according to claim 1, wherein the molar ratio of the amount of 2,6-octadien-1-ol produced to the amount of 2,7-octadien-1-ol produced is 0.01% to 2.0%.

3. The method for producing 2, 7-octadien-1-ol according to claim 1 or 2, wherein the molar ratio of the total amount of 1,3,7-octatriene and 1,7-octadien-3-ol produced to the amount of 2,7-octadien-1-ol produced is 1.0% to 20.0%.

4. The method for producing 2,7-octadien-1-ol according to any one of claims 1 to 3, wherein the Group 6 to 11 transition metal catalyst is a palladium compound.

5. The method for producing 2,7-octadien-1-ol according to claim 4, wherein a palladium concentration in the raw material mixture liquid is 10 to 10,000 ppm by mass.

6. The method for producing 2,7-octadien-1-ol according to any one of claims 1 to 5, wherein the tertiary phosphorus compound is an aromatic phosphine.

7. The method for producing 2,7-octadien-1-ol according to claim 6, wherein the aromatic phosphine is a hydrophilic aromatic phosphine.

8. The method for producing 2,7-octadien-1-ol according to claim 7, wherein the hydrophilic aromatic phosphine is at least one selected from the group consisting of lithium 3-diphenylphosphinobenzenesulfonate, triethylamine 3-diphenylphosphinobenzenesulfonate, and sodium 3-diphenylphosphinobenzenesulfonate.

9. The method for producing 2,7-octadien-1-ol according to any one of claims 1 to 8, wherein the temperature of the reaction liquid in the telomerization reactor is 50°C to 90°C.

10. The method for producing 2,7-octadien-1-ol according to any one of claims 1 to 9, wherein the raw material mixture liquid further contains an organic solvent, and the organic solvent is at least one selected from the group consisting of tetrahydrofuran, 1, 4-dioxane, and sulfolane.

11. The method for producing 2,7-octadien-1-ol according to any one of claims 1 to 10, wherein a content of the tertiary phosphorus compound in the raw material mixture liquid is 0.1 to 100 mol times relative to a content of the Group 6 to 11 transition metal.

12. The method for producing 2,7-octadien-1-ol according to claim 1, wherein the molar ratio of the amount of 2,6-octadien-1-ol produced to the amount of 2,7-octadien-1-ol produced is 0.01% to 2.0%, the Group 6 to 11 transition metal catalyst is a palladium compound, and a palladium concentration in the raw material mixture liquid is 10 to 10,000 ppm by mass.
